(19) Europäisches Patentamt
European Patent Office
Office européen des brevets

(11) **EP 3 755 230 B1**

(12) **EUROPEAN PATENT SPECIFICATION**

(45) Date of publication and mention
of the grant of the patent:
**24.05.2023  Bulletin 2023/21**

(21) Application number: **19704340.9**

(22) Date of filing: **18.02.2019**

(51) International Patent Classification (IPC):
*A61B 8/06* (2006.01)    *A61B 8/08* (2006.01)
*A61B 8/14* (2006.01)    *A61B 8/00* (2006.01)
*G01S 15/89* (2006.01)    *G01S 7/52* (2006.01)

(52) Cooperative Patent Classification (CPC):
**A61B 8/466; A61B 8/06; A61B 8/065;
A61B 8/0883; A61B 8/14; A61B 8/145;
A61B 8/469; A61B 8/483; A61B 8/488;
A61B 8/5223; G01S 7/52071; G01S 15/8927;
G01S 15/8979; G16H 50/30**

(86) International application number:
**PCT/EP2019/053920**

(87) International publication number:
**WO 2019/158741 (22.08.2019 Gazette 2019/34)**

(54) **METHOD AND APPARATUS FOR SIMULTANEOUS 4D ULTRAFAST BLOOD FLOW AND TISSUE DOPPLER IMAGING OF THE HEART AND RETRIEVING QUANTIFICATION PARAMETERS**

VERFAHREN UND VORRICHTUNG ZUR SIMULTANEN ULTRASCHNELLEN 4D-DOPPLER-BILDGEBUNG VON BLUTFLUSS UND GEWEBE DES HERZENS UND ZUR WIEDERHERSTELLUNG VON QUANTIFIZIERUNGSPARAMETERN

PROCÉDÉ ET APPAREIL D'ÉCOULEMENT DE SANG ULTRARAPIDE 4D SIMULTANÉ ET IMAGERIE DOPPLER TISSULAIRE DU COEUR ET D'EXTRACTION DE PARAMÈTRES DE QUANTIFICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.02.2018  EP 18305173**

(43) Date of publication of application:
**30.12.2020  Bulletin 2020/53**

(73) Proprietors:
• **INSERM (Institut National de la Santé et de la Recherche Médicale)**
**75013 Paris (FR)**
• **Ecole Supérieure de Physique et de Chimie Industrielles de la Ville de Paris**
**75005 Paris (FR)**
• **CENTRE NATIONAL DE LA RECHERCHE SCIENTIFIQUE -CNRS-**
**75016 Paris (FR)**
• **SORBONNE UNIVERSITE**
**75006 Paris (FR)**

• **Université Paris Cité**
**75006 Paris (FR)**

(72) Inventors:
• **PERNOT, Mathieu**
**75012 PARIS (FR)**
• **PAPADACCI, Clément**
**75012 PARIS (FR)**
• **TANTER, Mickael**
**75012 PARIS (FR)**

(74) Representative: **Plasseraud IP**
**66, rue de la Chaussée d'Antin**
**75440 Paris Cedex 09 (FR)**

(56) References cited:
WO-A1-2017/069699    US-A1- 2011 208 056
US-A1- 2013 144 161    US-A1- 2015 366 532
US-A1- 2016 140 730    US-A1- 2017 035 393

EP 3 755 230 B1

**Description**

FIELD

The disclosure relates to methods and apparatus for for simultaneous 4D ultrafast blood flow and tissue Doppler imaging of the heart and retrieving quantification parameters.

BACKGROUND

Echography ultrasound imaging is a portable, fast and low-cost technology that is routinely used in cardiology due to its ability to perform real-time imaging of the heart. Morphological parameters like cavity volumes and dynamic functional detection such as left ventricle outflow tract can be measured for diagnosis in two dimensions (2D) and one dimension (1D) respectively. Many more indexes used to characterize the state of the heart are measured routinely in real-time in one or two dimensions. The choice between 1D and 2D imaging is motivated by the frame-rate needed to measure physiological phenomena. To measure the cavity volume for instance, 2D imaging is more suitable as the frame rate needed to capture the global motion of the heart does not exceed real-time. However, to quantify fast physiological phenomena such as small tissue motion (E'/A' factor), blood flow speed (E/A factor) or both in the same time (E/E'), 1D imaging is performed to decrease the number of transmitted ultrasonic waves to allow a frame rate increase. Such routine exams take a noticeable amount of time due to manual selection of the region of interest. Moreover, such manual selections induce operator variability.

One advanced type of ultrasound imaging, ultrafast ultrasound imaging, has been largely studied [M. Tanter and M. Fink, "Ultrafast imaging in biomedical ultrasound," IEEE Trans. Ultrason. Ferroelectr. Freq. Control, in press, Jan. 2014] . It enables to increase the frame rate to reach few kilo images per second. The method relies on the emission of unfocused wave to insonifiate all the medium in few transmits. Recently, ultrafast imaging was extended to 4D ultrasound imaging, i.e. animated 3D ultrasound imaging. In particular, 4D ultrasound ultrafast imaging was performed to image blood flow in the left ventricle of human heart during a cardiac cycle, as well as blood flow and tissue motion of the carotid during a cardiac cycle [J. Provost et al., "3D ultrafast ultrasound imaging in vivo," Phys. Med. Biol., vol. 59, no. 19, p. L1, Oct. 2014.]

US2015366532A1, according to its abstract, states that a regurgitant orifice of a valve is detected. The valve is detected from ultrasound data. An anatomical model of the valve is fit to the ultrasound data. This anatomical model may be used in various ways to assist in valvular assessment. The model may define anatomical locations about which data is sampled for quantification. The model may assist in detection of the regurgitant orifice using both B-mode and color Doppler flow data with visualization without the jet. Segmentation of a regurgitant jet for the orifice may be constrained by the model. Dynamic information may be determined based on the modeling of the valve over time.

US2016140730A1, according to its abstract, states that a method of processing three-dimensional images or volumetric datasets to determine a configuration of a medium or a rate of a change of the medium, includes tracking changes of a field related to the medium to obtain a deformation or velocity field in three dimensions. In some cases, the field is a brightness field inherent to the medium or its motion. In other embodiments, the brightness field is from a tracking agent that includes floating particles detectable in the medium during flow of the medium.

US2013144161A1, according to its abstract, states that velocities are unaliased using conditional random fields. To constrain the energy minimization function, a global term includes a measure of a level of aliasing. In one example, the measure of the level of aliasing is based on a change in volume, such as the volume of the left ventricle. The unaliasing is performed along one or more surfaces, such as surfaces intersecting the mitral annulus and the left ventricle outflow tract. The anatomy used is identified and/or tracked using one or more machine-learnt detectors. Both B-mode and velocity information may be used for detecting the anatomy.

US2011208056A1, according to its abstract, states that volumetric quantification is provided in medical diagnostic ultrasound. By acquiring both B-mode and color flow data without stitching or acquiring in real-time at tens of volumes a second, more reliable quantification may be provided. Using multiple regions of interest in a volume may allow for more accurate and/or complete flow information, such as averaging flow from different locations in the same structure (e.g., use preservation of mass to acquire multiple measures of the same flow).

WO2017069699A1, according to its abstract, states a medical image processing method of assessing right ventricular function in a subject from medical image data. The medical image data comprising a sequence of images of the right ventricular region of the subject during a cardiac cycle for each of a plurality of views, each view of the plurality of views corresponding to a plane defined in relation to features of the right ventricular region. The method comprises: in each view of the plurality of views, tracking reference points in that view and determining the locations of the reference points in each image of the sequence of images corresponding to that view; and calculating a velocity of each reference point using the locations of that reference point in successive images of the sequence of images.

SUMMARY

Embodiments described therein provide for enhanced methods and apparatus for ultrasound imaging of the heart.

To this end, a method is provided as stated in claim 1.

Thanks to these dispositions, the quantification parameter is computed instantly, accurately in a repetitive manner, without any variability due the experience of the operator.

The method may further include one and / or other of the following features:

- during said acquisition step (a), said unfocused ultrasonic waves are transmitted in several series of successive unfocused ultrasonic waves, the successive unfocused ultrasonic waves of each series having respectively different propagation directions, and said imaging step (b) includes synthesizing a 3D image by ultrasound synthetic imaging from the respective raw data corresponding to said successive unfocused ultrasonic waves of each series;

- each series of successive unfocused ultrasonic waves includes 1 to 100 successive unfocused ultrasonic waves, for instance 1 to 20 successive unfocussed ultrasonic waves;

- said unfocused ultrasonic waves transmitted during said acquisition step (a) are divergent;

- during said acquisition step (a), said divergent ultrasonic waves have virtual sources behind the 2D array probe (i.e. opposite the direction of transmission of the waves);

- during said acquisition step, said unfocused ultrasonic waves are transmitted at a rate of more than 10 000 unfocused ultrasonic waves per second;

- the quantification parameter involves a peak blood velocity in a certain anatomic area and said locating step (d) includes automatically locating said anatomic area in the sequence of 3D images and automatically locating said point of interest as a point of maximum blood velocity in the sequence of 3D images;

- the quantification parameter involves a temporal variation such as acceleration time or deceleration time or time integral of the blood velocity in a certain anatomic area and said locating step (d) includes automatically locating said anatomic area in the sequence of 3D images and automatically locating said point of interest as a point of maximum blood velocity in the sequence of 3D images;

- the quantification parameter involves a blood flow rate or Cardiac Output (CO) obtained by the time and space integral of the blood velocity in a certain anatomic area (e.g. total blood flow-rate entering or leaving the heart according to the time in the cardiac cycle) and said locating step (d) includes automatically locating said anatomic area in the sequence of 3D images and automatically locating said point of interest as a point of maximum blood velocity in the sequence of 3D images;

- the quantification parameter involves a peak tissue velocity in a certain anatomic area and said locating step (d) includes automatically locating said anatomic area in the sequence of 3D images and automatically locating said point of interest as a point of maximum tissue velocity in said anatomic area in the sequence of 3D images ;

- the quantification parameter involves a tissue velocity at a certain anatomic position in the heart and said locating step (d) includes automatically locating said anatomic position in the sequence of 3D images;

- the quantification parameter determined at said quantification step is chosen from E, A, E', A', S, D, Vp, S', E/A, E/E', E/E', E'/A', S, S/D, Q, Qsystolic, Qdiastolic, DT, IVRT, PVAT, VTI, Gmean and Gmax wherein:

  - E is the early diastolic trans-mitral flow velocity;
  - E' is the early diastolic mitral annular velocity;
  - A is late diastolic trans-mitral flow velocity;
  - A' is the late diastolic mitral annular velocity;
  - S is the peak pulmonary venous systolic velocity;
  - D is the peak pulmonary venous early diastolic velocity;
  - Vp is the velocity of flow progression;
  - Q is the flow rate or Cardiac Output; Qsystolic is the total output transaortic flow rate and Qdiastolic is the total input transmitral flow rate;
  - DT is the e-wave deceleration time;
  - PVAT is the pulmonary acceleration time;
  - IVRT is the length of the isovolumetric relaxation time;
  - Gmean and Gmax are the mean and maximum transvalvular pressure gradients;
  - VTI is velocity time integral;
  - S' is the peak systolic annular velocity;

- during said acquisition step, said unfocussed ultrasonic waves are transmitted at a rate of more than 10 000 unfocussed ultrasonic waves per second;

- during said acquisition step (a), said unfocussed ultrasound waves are transmitted in the heart for a duration of at least part of a cardiac cycle and less than 10 cardiac cycles, for instance less than 5 cardiac cycles;
- during said acquisition step (a), said unfocussed ultrasound waves are transmitted in the heart for a duration comprised between 1s and 10s, for instance between 1s and 5s.

Besides, an apparatus for 4D imaging of a heart of a living being is provided as stated in claim 7.

The apparatus may further include one and / or other of the following features:

- the quantification parameter involves a peak blood velocity in a certain anatomic area and said control system is configured to automatically locate said anatomic area in the sequence of 3D images and to automatically locate said point of interest as a point of maximum blood velocity in the sequence of 3D images;
- the quantification parameter involves a peak tissue velocity in a certain anatomic area and said control system (is configured to automatically locate said anatomic area in the sequence of 3D images and to automatically locate said point of interest as a point of maximum tissue velocity in said anatomic area in the sequence of 3D images;

- the quantification parameter involves a temporal variation such as acceleration time or deceleration time or time integral of the blood velocity in a certain anatomic area and said locating step (d) includes automatically locating said anatomic area in the sequence of 3D images and automatically locating said point of interest as a point of maximum blood velocity in the sequence of 3D images;
- the quantification parameter involves a blood flow rate obtained by the time and space integral of the blood velocity in a certain anatomic area (e.g. total blood flow-rate entering or leaving the heart according to the time in the cardiac cycle) and said locating step (d) includes automatically locating said anatomic area in the sequence of 3D images and automatically locating said point of interest as a point of maximum blood velocity in the sequence of 3D images;
- the quantification parameter involves a tissue velocity at a certain anatomic position in the heart and said control system is configured to automatically locate said point of interest at said anatomic position in the sequence of 3D images;
- the quantification parameter is chosen from E, A, E', A', S, D, Vp, S', E/A, E/E', E/E', E'/A', S, S/D, Q, Qsystolic, Qdiastolic, DT, IVRT, PVAT, VTI, Gmean and Gmax wherein:

  - E is the early diastolic trans-mitral flow velocity;
  - E' is the early diastolic mitral annular velocity;
  - A is late diastolic trans-mitral flow velocity;

  - A' is the late diastolic mitral annular velocity;
  - S is the peak pulmonary venous systolic velocity;
  - D is the peak pulmonary venous early diastolic velocity;
  - Vp is the velocity of flow progression;
  - Q is the flow rate or cardiac output;
  - Qsystolic is the total output transaortic flow rate;
  - Qdiastolic is the total input transmitral flow rate;
  - DT is the e-wave deceleration time;
  - PVAT is the pulmonary acceleration time;
  - IVRT is the length of the isovolumetric relaxation time;
  - Gmean and Gmax are the mean and maximum transvalvular pressure gradients;
  - VTI is velocity time integral;
  - S' is the peak systolic annular velocity;

- said control system is configured to transmit said unfocussed ultrasonic waves in several series of successive unfocussed ultrasonic waves, the successive unfocussed ultrasonic waves of each series having respectively different propagation directions, and said control system is configured to synthesize a 3D image by ultrasound synthetic imaging from the respective raw data corresponding to said successive unfocussed ultrasonic waves of each series;
- each series of successive unfocused ultrasonic waves includes 1 to 100 successive unfocused ultrasonic waves, for instance 1 to 20 successive unfocussed ultrasonic waves;
- said control system (3, 4) is configured to transmit said unfocused ultrasonic waves as divergent ultrasonic waves;
- said divergent ultrasonic waves have virtual sources behind the 2D array probe (i.e. opposite the direction of transmission of the waves);
- said control system is configured to transmit said unfocussed ultrasonic waves at a rate of more than 10 000 unfocussed ultrasonic waves per second;

- said control system is configured to transmit said unfocussed ultrasound waves for a duration of at least part of a cardiac cycle and less than 10 cardiac cycles, for instance less than 5 cardiac cycles;
- said control system is configured to transmit said unfocused ultrasound waves for a duration comprised between 1s and 10s, for instance between 1s and 5s.

BRIEF DESCRIPTION OF THE DRAWINGS

Other features and advantages of the disclosure appear from the following detailed description of one nonlimiting example thereof, with reference to the accompanying drawings.
In the drawings:

- Figure 1 is a schematic drawing showing an apparatus for 4D imaging of the heart;
- Figure 2 is a block diagram showing part of the apparatus of Figure 1;
- Figure 3 is a diagram illustrating virtual sources of divergent ultrasound waves, generated by the apparatus of Figures 1-2;
- Figure 4 illustrates the transmission of a divergent ultrasound wave in the heart of a living being by the apparatus of Figures 1-2;
- Figure 5 illustrates the transmission of two successive divergent ultrasound waves with different directions of propagation, respectively from two virtual sources;
- Figure 6 illustrates results obtained simultaneous 4D ultrafast blood flow and 4D tissue velocity of the left ventricle of a human sound volunteer in a single heartbeat:

  - Figure 6(a) shows 5 cross sections of the left ventricle extracted from the sequence of 3D images generated by the apparatus, respectively allowing visualization of the cardiac phases (rapid inflow, diastasis, atrial systole, pre-ejection, ejection) which are separated by dotted lines on Figures 6(a)-6(d);
  - Figure 6(b) is a Doppler spectrogram of blood flow at the mitral valve;
  - Figure 6(c) is a tissue velocity curve at the basal septum location; and
  - Figure 6(d) is a corresponding electrocardiogram (ECG);

- Figure 7 illustrates images and measurements of the left ventricle, made by a trained operator with a classical 2D clinical ultrasound system for the same volunteer as that of Figure 6, with indication of cardiac phases as in Figure 6:
- Figure 7(a) shows the Doppler spectrum of blood flow at the mitral valve and
- Figure 7(b) shows tissue velocity at the basal septum obtained with a clinical ultrasound system for the healthy volunteer; and
- Figures 8 and 9 are respectively similar to Figures 6-7, for a patient with a hypertrophic cardiomyopathy.

MORE DETAILED DESCRIPTION

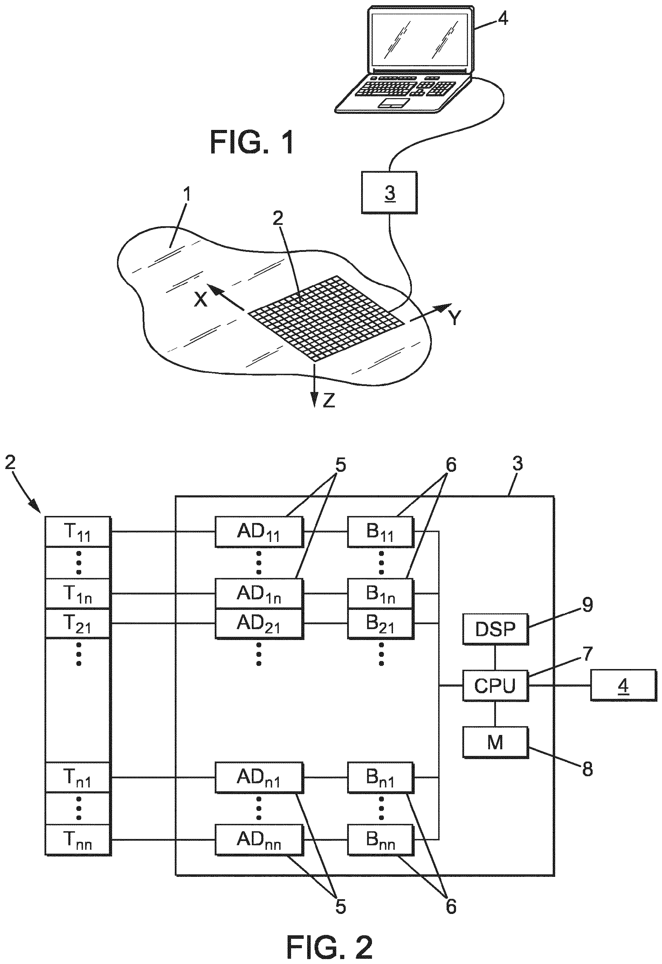

In the Figures, the same references denote identical or similar elements.
The apparatus shown on Figures 1 and 2 is adapted to ultrafast 4D ultrasound imaging of the heart of a living being 1, for instance a mammal and in particular a human.
The apparatus may include for instance at least a 2D array ultrasonic probe 2 and a control system.
The 2D array ultrasonic probe 2 may have for instance a few hundreds to a few thousands transducer elements $T_{ij}$, with a pitch lower than 1mm. The 2D array ultrasonic probe 2 may have n*n transducer elements disposed as a matrix along two perpendicular axes X, Y, transmitting ultrasound waves along an axis Z which is perpendicular to the XY plane. In one specific example, the 2D array ultrasonic probe 2 may have 1024 transducer elements $T_{ij}$ (32*32), with a 0.3 mm pitch. The transducer elements may transmit for instance at a central frequency comprised between 1 and 10 MHz, for instance of 3 MHz.
The control system may for instance include a specific control unit 3 and a computer 4. In this example, the control unit 3 is used for controlling 2D array ultrasonic probe 2 and acquiring signals therefrom, while the computer 4 is used for controlling the control unit 3, generating 3D image sequences from the signals acquired by control unit 3 and determining quantification parameters therefrom. In a variant, a single electronic device could fulfill all the functionalities of control unit 3 and computer 4.
As shown on Figure 2, control unit 3 may include for instance:

- n*n analog/digital converters 5 ($AD_{ij}$) individually connected to the n transducers $T_{ij}$ of 2D array ultrasonic probe 2;
- n*n buffer memories 6 ($B_{ij}$) respectively connected to the n*n analog/digital converters 5;

- a central processing unit 7 (CPU) communicating with the buffer memories 6 and the computer 4;
- a memory 8 (MEM) connected to the central processing unit 7;
- a digital signal processor 9 (DSP) connected to the central processing unit 7.

The apparatus may operate as follows.

**(a) Acquisition:**

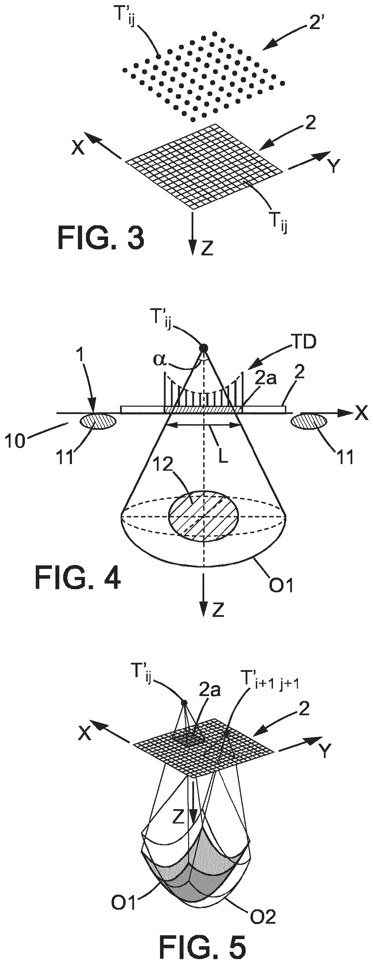

The 2D array ultrasonic probe 2 is placed on the chest 10 of the patient 1, usually between two ribs, in front of the heart 12 of the patient as shown in Figure 4.

Because of the limited intercostal space between ribs 11 compared to the size of the heart 12 to be imaged, the 2D array ultrasonic probe 2 is controlled to transmit divergent ultrasonic waves in the chest 10, for instance spherical ultrasonic waves (i.e. having a spherical wave front O1). The control system may be programmed such that the ultrasonic waves are transmitted at a rate of several thousand ultrasonic waves per second, for instance more than 10 000 unfocussed ultrasonic waves per second.

Spherical waves can be generated by a single transducer element (with low amplitude) or more advantageously with higher amplitude by a large part of the matrix array using one or more virtual point sources $T'_{ij}$ forming a virtual array 2' placed behind of in front of the 2D array ultrasonic probe 2, as shown in Figures 3-4. The transmit delay TD applied by the control system to a transducer element e placed in position $\begin{pmatrix} x_e \\ y_e \\ 0 \end{pmatrix}$ associated to the virtual source v placed in position $\begin{pmatrix} x_v \\ y_v \\ z_v \end{pmatrix}$ is:

$$TD = \sqrt{z_v^2 + (x_e - x_v)^2 + (y_e - y_v)^2}/c$$

where c is the speed of sound.

For each virtual source $T'_{ij}$ used, it is possible for the control system to activate only a subset 2a of the 2D array ultrasonic probe 2, having a sub-aperture L which determines the aperture angle $\alpha$ of the divergent ultrasonic wave. The aperture angle $\alpha$ may be for instance of 90°. The imaged depth along axis Z may be about 12 to 15 cm.

It is possible to use only one virtual source $T'_{ij}$ and thus one ultrasonic wave for each 3D image of the heart, as will be explained later.

However, to enhance image resolution and contrast, it is useful to transmit the unfocused ultrasonic waves in series of successive unfocussed ultrasonic waves, the successive unfocused ultrasonic waves of each series having respectively different propagation directions: in that case, each 3D image is synthesized from the signals acquired from one of said series of successive unfocussed ultrasonic waves as will be explained later. The successive ultrasonic waves of each series may be obtained by varying the virtual source $T_{ij}$ from one wave to the other, thus varying the wave front O1, O2 etc., as shown in Figure 5. Each series may include for instance 5 to 20 successive ultrasonic waves of different directions, for instance 10 to 20 successive ultrasonic waves of different directions.

In all cases, after each ultrasonic wave is transmitted, backscattered echoes are acquired by said 2D array ultrasonic probe (sampled for instance with a sampling rate of 12 MHz) and memorized. This raw data (also usually called RF data or radiofrequency data) is the used to generate a sequence of 3D images.

The duration of acquisition may be comprised between 10ms and and a few cardiac cycles, for instance at least one part of the cardiac cycle (for instance the diastole or systole, or one cardiac cycle) and less than 10 cardiac cycles (for instance less than 5 cardiac cycles). Such duration may be for instance comprised between 1s and 10s (for instance less than 5s). In a specific example, such duration is around 1.5s.

An electrocardiogram (ECG) may be co-recorded during the acquisition.

**(b) Imaging:**

After receiving the backscattered echoes, a parallel beamforming may be directly applied by the control system to reconstruct the 3D image from each single ultrasonic wave. Delay and sum beamforming can be used in the time domain or in the Fourier domain. In the time domain, the delays applied on the signal received by each transducer element e to reconstruct a voxel placed in $\begin{pmatrix} x \\ y \\ z \end{pmatrix}$ is the sum of the forward propagation time from the virtual source v to the voxel and the backscattered propagation time to the transducer element e:

$$Delay = forward\ delay + Backscattered\ delay$$

$$Forward\ delay = \sqrt{(z-z_v)^2 + (x-x_v)^2 + (y-y_v)^2}/c$$

$$Backscattered\ delay = \sqrt{z^2 + (x_e-x)^2 + (y_e-y)^2}/c$$

In case the ultrasonic waves are transmitted by series of ultrasonic waves having respectively different propagation directions as explained above, each image can be obtained by the control system through known processes of synthetic imaging. Voxels are beamformed using delay- and-sum algorithms for each virtual source and subsequently coherently compounded to form a final, high quality 3D image. Details of such synthetic imaging can be found for instance in:

Montaldo, G., Tanter, M., Bercoff, J., Benech, N., Fink, M., 2009. Coherent plane-wave compounding for very high frame rate ultrasonography and transient elastography. IEEE Trans. Ultrason. Ferroelectr. Freq. Control 56, 489-506. doi:10.1109/TUFFC.2009.1067

Nikolov, S.I., 2001. Synthetic aperture tissue and flow ultrasound imaging. Orsted-DTU, Technical University of Denmark, Lyngby, Denmark.

Nikolov, S.I., Kortbek, J., Jensen, J.A., 2010. Practical applications of synthetic aperture imaging, in: 2010 IEEE Ultrasonics Symposium (IUS). Presented at the 2010 IEEE Ultrasonics Symposium (IUS), pp. 350-358. doi : 10.1109/ULTSYM. 2010.593562 7

Lockwood, G.R., Talman, J.R., Brunke, S.S., 1998. Real-time 3-D ultrasound imaging using sparse synthetic aperture beamforming. IEEE Trans. Ultrason. Ferroelectr. Freq. Control 45, 980-988. doi:10.1109/58.710573

Papadacci, C., Pernot, M., Couade, M., Fink, M. & Tanter, M. High-contrast ultrafast imaging of the heart. IEEE transactions on ultrasonics, ferroelectrics, and frequency control 61, 288-301, doi:10.1109/tuffc.2014.6722614 (2014).

The framerate, i.e. the rate of 3D images in the animated sequence which is finally obtained, may be of several thousand 3D images per second, for instance 3000 to 5000 3D images per second.

**(c)Blood and tissue velocity computing:**

Blood flow and tissue motion estimation may be performed by the control system using known methods.

For instance, the Kasai algorithm may be used to estimate motion in blood and in tissues with a half-wavelength spatial sampling (Kasai, C., Namekawa, K., Koyano, A., Omoto, R., 1985. Real-Time Two-Dimensional Blood Flow Imaging Using an Autocorrelation Technique. IEEE Trans. Sonics Ultrason. 32, 458-464. doi:10.1109/T-SU.1985.31615). Blood flow can be estimated by first applying a high-pass filter to the baseband data and then, for each individual voxel, Power Doppler may be obtained by integrating the power-spectral density, Pulsed Doppler may be obtained by computing the short-time Fourier transform, and Color Doppler maps may be obtained by estimating the first moment of the voxel-specific Pulsed-Doppler spectrogram. Power velocity integral maps can be obtained by computing the time integral of power times velocity in order to obtain images of a parameter related to flow rate. Advanced filtering such as Spatio-temporal filters based on singular value decomposition can also be used to better remove the clutter signal (Demené, C. et al. Spatiotemporal Clutter Filtering of Ultrafast Ultrasound Data Highly Increases Doppler and Ultrasound Sensitivity. IEEE transactions on medical imaging 34, 2271-2285, doi:10.1109/tmi.2015.2428634 (2015)).

In a specific example:

- 4D tissue velocity may be computed by performing 1D cross-correlation to obtain volumes of tissue volume-to-volume axial displacements. A butter-worth low-pass filtering with a 60Hz cut-off frequency was then applied on the displacements. A myocardium 3D mask (specific to the tissues of the myocardium) may be applied to remove signal outside the muscle. To display 4D tissue velocities, Amira® software may be used. In each voxel, one tissue velocity curve may be derived.

- 4D Color Doppler may be computed by performing an SVD filtering to remove signal from the tissue and keep only the signal from the blood flow as it is done for instance in the above publication by Demené et al. 1D axial cross-correlation pixel-per-pixel on SVD-filtered voxels may be performed to obtain Color Doppler volumes. To display 4D Color Doppler, a cavity 3D mask (specific to the cavity receiving blood, e.g. the left ventricular cavity) may be applied to remove signal outside the cavity and the volume rendering may be performed using Amira® software.

The above-mentioned 3D masks of the left ventricle cavity and myocardium may be computed as follows. The cavity may be segmented using Power Doppler flow integrated over the entire cardiac cycle on the 3D images. The myocardium may be segmented using integrated tissue velocity over the cardiac cycle and manual selection of the contour on two perpendicular 2D slices. An elliptic interpolation may be used to get the three-dimensional representation.

More generally, step (c) involves automatically computing 3D cartography of at least one parameter related to blood velocity and / or tissue velocity in said imaged volume, based on said sequence of 3D images. Said 3D cartography may consist of an animated sequence of 3D images of the computed parameter. The parameter may be blood and / or tissue velocity, or a component thereof.

### (d) Locating of points of interest:

Depending on the quantification parameters which are sought, at least one point of interest having a predetermined property is automatically located by the control system in the sequence of 3D images.

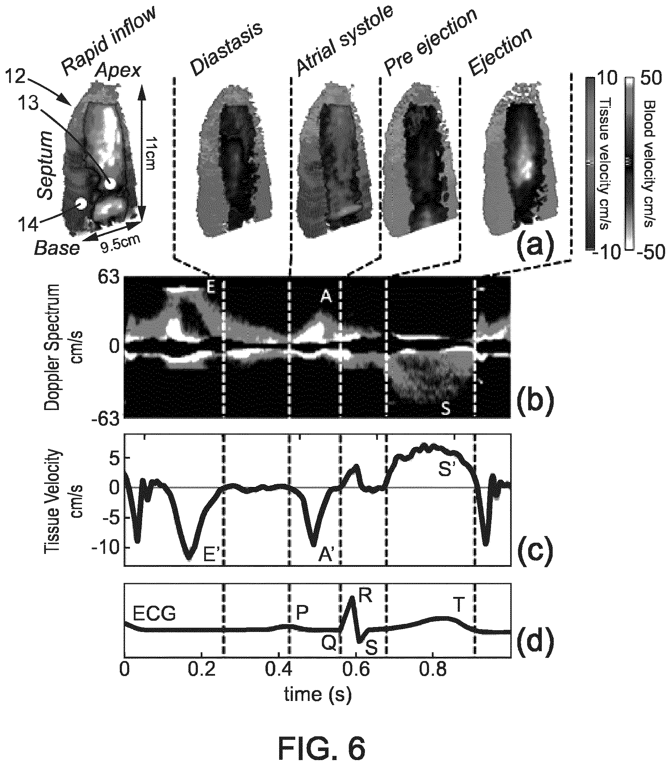

When the quantification parameter involves a peak blood velocity in a certain anatomic area, the control system may automatically locate said point of interest as a point of maximum blood velocity in said anatomic area and in at least part of the sequence of 3D images. For instance, when the early diastolic trans-mitral flow velocity E has to be computed, i.e. the peak blood velocity through the mitral valve during the E wave of the cardiac cycle (early diastolic wave), the control system (and more particularly computer 4) may automatically spot the point 13 (Figure 6a) of peak blood velocity inside the mitral valve. In a specific example, a Fourier transform over time may be performed at each voxel using a 60 sample sliding window to retrieve a spectrogram everywhere in the volume. Automatic dealiasing may be performed according to the above Demené et al. The location of point 13 may then be automatically detected by detecting the blood flow maximum.

When the quantification parameter involves tissue velocity at a certain anatomic position in the heart, the control system may automatically locate said anatomic position in the sequence of 3D images. For instance, when the early diastolic mitral annular velocity E' has to be computed (i.e. the velocity of the mitral valve during the E wave of the cardiac cycle) the control system (and more particularly computer 4) may automatically spot a point 14 of the mitral valve (Figure 6a). Such automatic location may be done according to an anatomic model of the heart memorized in computer 4, or by selecting a point in the tissues in correspondence with the above point 14 of maximum blood velocity.

When the quantification parameter involves a peak tissue velocity in a certain anatomic area, the control system may automatically locate said anatomic area in the sequence of 3D images and said point of interest as a point of maximum tissue velocity in said anatomic area in the sequence of 3D images. For instance, when the peak systolic annular velocity S' of the left ventricle has to be computed, the system determines a point (not shown) of the tissues surrounding the ventricle having the maximum velocity in the image sequence myocardium.

### (e) Quantification

The desired quantification parameter(s) can then be computed by the control system (and in particular by computer 4) based on the previously determined point(s) of interest, and based on the peak blood or tissue velocity of such point of interest.

Particularly useful examples of such quantification parameters are E, A, E', A', S, D, Vp, S', E/A, E/E', E/E', E'/A', S, S/D, Q, Qsystolic, Qdiastolic, DT, IVRT, PVAT, VTI, Gmean and Gmax wherein:

- E is the early diastolic trans-mitral flow velocity as defined above;
- E' is the early diastolic mitral annular velocity as defined above (computed at the instant of peak blood velocity corresponding to E);
- A is late diastolic trans-mitral flow velocity;
- A' is the late diastolic mitral annular velocity (computed at the instant of peak blood velocity corresponding to A);
- S is the peak pulmonary venous systolic velocity;
- D is the peak pulmonary venous early diastolic velocity;
- Vp is the velocity of flow progression;

- Q is the flow rate or cardiac output;
- Qsystolic is the total output transaortic flow rate;
- Qdiastolic is the total input transmitral flow rate;
- DT is the e-wave deceleration time;
- PVAT is the pulmonary acceleration time;
- IVRT is the length of the isovolumetric relaxation time;
- Gmean and Gmax are the mean and maximum transvalvular pressure gradients;
- VTI is velocity time integral;
- S' is the peak systolic annular velocity as defined above.

It should be noted that:

- at step (d), said at least one point of interest is automatically located based solely on said 3D cartography and its temporal profile;
- and at step (e), said at least one velocity is automatically determined at said at least one point of interest based solely on said 3D cartography and its temporal profile.

More generally, in the present disclosure the transvalvular blood flows can be localized using only the spatial and temporal velocity information without any additional anatomic information. Temporal profiles of the flow velocity (or Spectral Doppler profile) are indeed a strong characteristic of the valve location and are very specific to the type of valve:

- Transaortic blood flow is characterized by a strong outflow during the entire systole, followed by no flow (or lower flow in the reverse direction in case of aortic regurgitation) during the diastole. The transaortic flow can then be localized precisely by determining the spatial peak of the outflow blood velocity.
- In contrast, transmitral blood flow is characterized by no or little flow in systole and two inflow peaks in early and late diastole. The transmitral flow can then be localized precisely by finding the spatial peak of the inflow blood velocity.

This is made possible in particular because the temporal profiles are obtained simultaneously at every voxel of the image.

The point and interest and the velocity at this point of interest are thus determined without need of anatomical image, in particular without need of a B-mode anatomical image, thanks to the fact that the present method involves determining the 3D cartography of velocity in the whole imaged volume. Thus, the whole method of the present disclosure needs no B-mode imaging, and more generally no anatomical imaging, which enables quicker results of the present method.

**Example:**

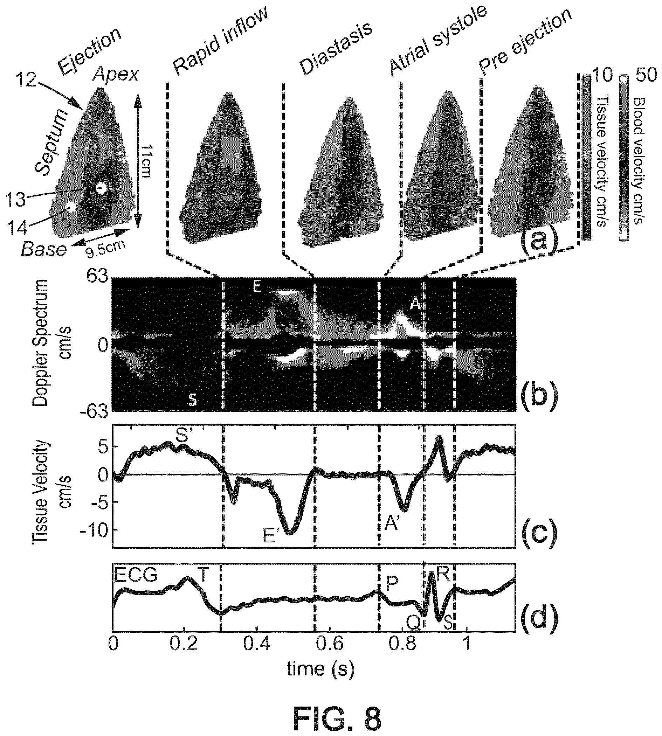

The left ventricle of a healthy human volunteer and a young patient with a hypertrophic cardiomyopathy were imaged (respectively Figures 6 and 8) and the index E/E' was automatically computed for both cases.

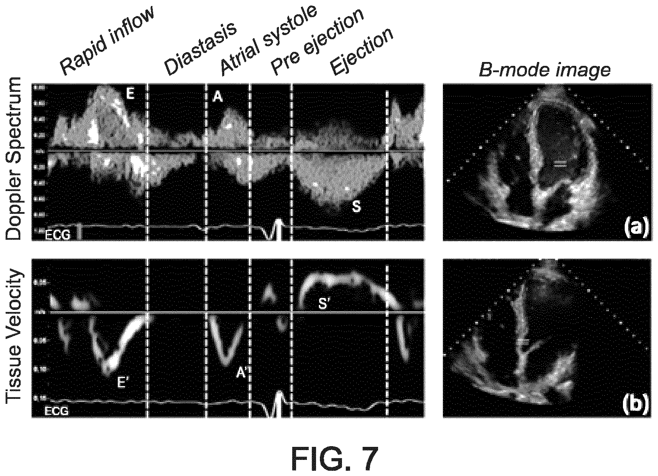

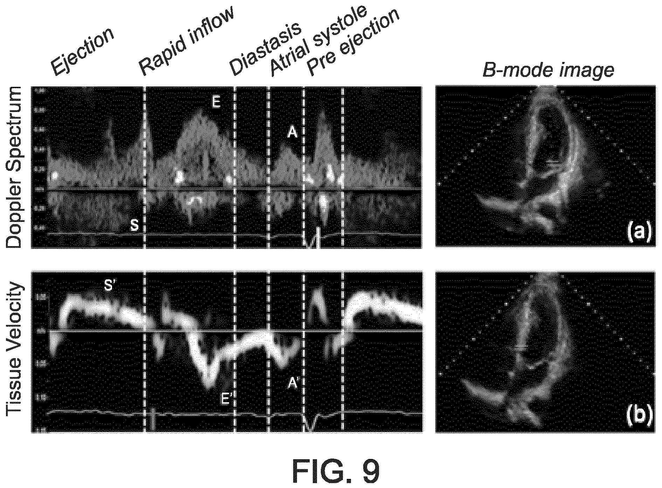

The healthy human volunteer and the young patient were then scanned by a cardiologist using a classical clinical ultrasound system on the apical 4-chambers view (Figures 7 and 9). Doppler spectrum and tissue velocity were assessed using pulsed Doppler and tissue Doppler modes and the index E/E' was automatically computed for both cases. These manual measures confirmed the accuracy of the automatic measurement, while the automatic measurement is much quicker (it is done in one heartbeat) and does not require specific training for the operator.

**Claims**

1. Method for 4D imaging of a heart of a living being, said method including at least the following steps:

   (a) an acquisition step wherein unfocused ultrasonic waves are transmitted in the heart by a 2D array ultrasonic probe (2) and raw data from backscattered ultrasonic waves are acquired by said 2D array ultrasonic probe (2);
   (b) an imaging step wherein a sequence of 3D images is generated from said raw data, said sequence of 3D images forming an animation showing movements of an imaged volume including at least part of the heart;
   (c) a velocity computing step wherein 3D cartography of at least one parameter related to blood velocity and tissue velocity are automatically computed in said imaged volume and time, based on said sequence of 3D images;
   (d) a locating step wherein at least one point of interest having a predetermined property is automatically located in said sequence of 3D images, wherein said at least one point of interest is automatically located based solely on said 3D cartography and its temporal profile;

(e) a quantification step wherein at least one velocity chosen between blood velocity and tissue velocity is automatically determined at said at least one point of interest and a predetermined quantification parameter is automatically computed, involving said at least one velocity, wherein said at least one velocity is automatically determined at said at least one point of interest based solely on said 3D cartography and its temporal profile;

wherein the quantification parameter involves :

- either a peak blood velocity in a certain anatomic area and said locating step (d) includes automatically locating said point of interest (13) as a point of maximum blood velocity in said anatomic area and in at least part of the sequence of 3D images;
- or a peak tissue velocity in a certain anatomic area and said locating step (d) includes automatically locating said anatomic area in the sequence of 3D images and automatically locating said point of interest as a point of maximum tissue velocity in said anatomic area in the sequence of 3D images.

2. Method according to claim 1, wherein the quantification parameter involves a temporal variation of the parameter related to the velocity in a certain anatomic area and said locating step (d) includes automatically locating said point of interest (13) as a point of maximum blood velocity in said anatomic area and in at least part of the sequence of 3D images.

3. Method according to claim 1, wherein the quantification parameter involves a time integral of the parameter related to the velocity in a certain anatomic area and said locating step (d) includes automatically locating said point of interest (13) as a point of maximum blood velocity in said anatomic area and in at least part of the sequence of 3D images.

4. Method according to claim 1, wherein the quantification parameter involves a space integral of the parameter related to the velocity in a certain anatomic area and said locating step (d) includes automatically locating said point of interest (13) as a point of maximum blood velocity in said anatomic area and in at least part of the sequence of 3D images.

5. Method according to any one of the preceding claims, wherein the quantification parameter determined at said quantification step is chosen from E, A, E', A', S, D, Vp, S', E/A, E/E', E/E', E'/A', S, S/D, VTI, Gmean and Gmax wherein:

   - E is the early diastolic trans-mitral flow velocity;
   - E' is the early diastolic mitral annular velocity;
   - A is late diastolic trans-mitral flow velocity;
   - A' is the late diastolic mitral annular velocity;
   - S is the peak pulmonary venous systolic velocity;
   - D is the peak pulmonary venous early diastolic velocity;
   - Vp is the velocity of flow progression;
   - Gmean and Gmax are the mean and maximum transvalvular pressure gradients;
   - VTI is velocity time integral;
   - S' is the peak systolic annular velocity.

6. Method according to any one of the preceding claims, wherein during said acquisition step (a), said unfocused ultrasonic waves are transmitted in several series of successive unfocused ultrasonic waves, the successive unfocused ultrasonic waves of each series having respectively different propagation directions, and said imaging step (b) includes synthesizing a 3D image by ultrasound synthetic imaging from the respective raw data corresponding to said successive unfocussed ultrasonic waves of each series.

7. Apparatus for 4D imaging of a heart of a living being, said apparatus including at least a 2D array ultrasonic probe (2) and a control system (3, 4) configured to:

   (a) transmit unfocussed ultrasonic waves in the heart through said 2D array ultrasonic probe (2) and acquire raw data from backscattered ultrasonic waves through said 2D array ultrasonic probe (2),
   (b) generate a sequence of 3D images from said raw data, said sequence of 3D images forming an animation showing movements of an imaged volume including at least part of the heart,

(c) automatically compute 3D cartography of at least one parameter related to blood velocity and tissue velocity in said imaged volume, based on said sequence of 3D images,

(d) automatically locate at least one point of interest (13, 14) having a predetermined property in said sequence of 3D images, wherein said control system (3, 4) is configured to automatically locate said at least one point of interest based solely on said 3D cartography and its temporal profile;

(e) automatically determine at least one velocity chosen between blood velocity and tissue velocity at said at least one point of interest (13, 14) and automatically compute a predetermined quantification parameter involving said at least one velocity, wherein said control system (3, 4) is configured to automatically determine said at least one velocity at said at least one point of interest based solely on said 3D cartography and its temporal profile, wherein the quantification parameter involves :

- either a peak blood velocity in a certain anatomic area and said control system (3, 4) configured to automatically locate said point of interest (13) as a point of maximum blood velocity in said anatomic area and in at least part of the sequence of 3D images;
- or a peak tissue velocity in a certain anatomic area and said control system (3, 4) configured to automatically locate said anatomic area in the sequence of 3D images and automatically locating said point of interest as a point of maximum tissue velocity in said anatomic area in the sequence of 3D images.

8. Apparatus according to claim 7, wherein the quantification parameter is chosen from E, A, E', A', S, D, Vp, S', E/A, E/E', E/E', E'/A', S, S/D, VTI, Gmean and Gmax wherein:

- E is the early diastolic trans-mitral flow velocity;
- E' is the early diastolic mitral annular velocity;
- A is late diastolic trans-mitral flow velocity;
- A' is the late diastolic mitral annular velocity;
- S is the peak pulmonary venous systolic velocity;
- D is the peak pulmonary venous early diastolic velocity;
- Vp is the velocity of flow progression;
- Gmean and Gmax are the mean and maximum transvalvular pressure gradients;
- VTI is velocity time integral;
- S' is the peak systolic annular velocity.

9. Apparatus according to any of claims 7-8, wherein said control system (3, 4) is configured to transmit said unfocussed ultrasonic waves in several series of successive unfocussed ultrasonic waves, the successive unfocussed ultrasonic waves of each series having respectively different propagation directions, and said control system (3, 4) is configured to synthesize a 3D image by ultrasound synthetic imaging from the respective raw data corresponding to said successive unfocussed ultrasonic waves of each series;

10. Apparatus according to any of claims 7-9, wherein said control system (3, 4) is configured to transmit said unfocussed ultrasonic waves as divergent ultrasonic waves.

11. Apparatus according to any of claims 7-10, wherein said control system (3, 4) is configured to transmit said unfocussed ultrasound waves for a duration of at least part of a cardiac cycle and less than 10 cardiac cycles.

12. Apparatus according to any of claims 7-11, wherein said control system (3, 4) is configured to transmit said unfocussed ultrasound waves for a duration comprised between 1s and 10s.

## Patentansprüche

1. Verfahren zur 4D-Abbildung eines Herzens eines Lebewesens, wobei das Verfahren wenigstens die folgenden Schritte umfasst:

(a) einen Erfassungsschritt, bei dem unfokussierte Ultraschallwellen im Herzen durch eine 2D-Array-Ultraschallsonde (2) übertragen werden und Rohdaten von rückgestreuten Ultraschallwellen durch die 2D-Array-Ultraschallsonde (2) erfasst werden;

(b) einen Bildgebungsschritt, bei dem eine Sequenz von 3D-Bildern aus den Rohdaten erzeugt wird, wobei die Sequenz von 3D-Bildern eine Animation bildet, die Bewegungen eines abgebildeten Volumens zeigt, das wenigstens einen Teil des Herzens umfasst;

(c) einen Geschwindigkeitsberechnungsschritt, bei dem eine 3D-Kartographie wenigstens eines Parameters, der mit der Blutgeschwindigkeit und der Gewebegeschwindigkeit zusammenhängt, in dem abgebildeten Volumen und der Zeit automatisch berechnet wird, basierend auf der Sequenz von 3D-Bildern;

(d) einen Lokalisierungsschritt, bei dem wenigstens ein betrachteter Punkt mit einer vorbestimmten Eigenschaft in der Sequenz von 3D-Bildern automatisch lokalisiert wird, wobei der wenigstens eine betrachtete Punkt ausschließlich auf der Grundlage der 3D-Kartographie und des zeitlichen Profils automatisch lokalisiert wird;

(e) einen Quantifizierungsschritt, bei dem wenigstens eine Geschwindigkeit, die aus der Blutgeschwindigkeit und der Gewebegeschwindigkeit gewählt wird, automatisch an dem wenigstens einen betrachteten Punkt bestimmt wird und ein vorbestimmter Quantifizierungsparameter automatisch berechnet wird, der die wenigstens eine Geschwindigkeit einbezieht, wobei die wenigstens eine Geschwindigkeit an dem wenigstens einen betrachteten Punkt ausschließlich basierend auf der 3D-Kartographie und ihrem zeitlichen Profil automatisch bestimmt wird; wobei der Quantifizierungsparameter umfasst:

- entweder eine Spitzengeschwindigkeit des Blutes in einem bestimmten anatomischen Bereich, und der Lokalisierungsschritt (d) beinhaltet das automatische Lokalisieren des betrachteten Punktes (13) als einen Punkt maximaler Blutgeschwindigkeit in dem anatomischen Bereich und in wenigstens einem Teil der Sequenz von 3D-Bildern;

- oder eine maximale Spitzengeschwindigkeit des Gewebes in einem bestimmten anatomischen Bereich und der Lokalisierungsschritt (d) beinhaltet das automatische Lokalisieren des anatomischen Bereichs in der Sequenz von 3D-Bildern und das automatische Lokalisieren des betrachteten Punkts als einen Punkt mit maximaler Gewebegeschwindigkeit in dem anatomischen Bereich in der Sequenz von 3D-Bildern.

2. Verfahren nach Anspruch 1, wobei der Quantifizierungsparameter eine zeitliche Veränderung des Parameters einbezieht, der mit der Geschwindigkeit in einem bestimmten anatomischen Bereich zusammenhängt, und der Lokalisierungsschritt (d) das automatische Lokalisieren des betrachteten Punktes (13) als einen Punkt maximaler Blutgeschwindigkeit in dem anatomischen Bereich und in wenigstens einem Teil der Sequenz von 3D-Bildern umfasst.

3. Verfahren nach Anspruch 1, wobei der Quantifizierungsparameter ein Zeitintegral des Parameters beinhaltet, der sich auf die Geschwindigkeit in einem bestimmten anatomischen Bereich bezieht, und der Lokalisierungsschritt (d) das automatische Lokalisieren des betrachteten Punktes (13) als einen Punkt maximaler Blutgeschwindigkeit in dem anatomischen Bereich und in wenigstens einem Teil der Sequenz von 3D-Bildern beinhaltet.

4. Verfahren nach Anspruch 1, wobei der Quantifizierungsparameter ein Raumintegral des Parameters einbezieht, der sich auf die Geschwindigkeit in einem bestimmten anatomischen Bereich bezieht, und der Lokalisierungsschritt (d) das automatische Lokalisieren des betrachteten Punktes (13) als einen Punkt maximaler Blutgeschwindigkeit in dem anatomischen Bereich und in wenigstens einem Teil der Sequenz von 3D-Bildern umfasst.

5. Verfahren nach einem der vorhergehenden Ansprüche, wobei der bei dem Quantifizierungsschritt bestimmte Quantifizierungsparameter gewählt ist aus E, A, E', A', S, D, Vp, S', E/A, E/E', E/E', E'/A', S, S/D, VTI, Gmean und Gmax, wobei:

- E die frühe diastolische transmitrale Fließgeschwindigkeit ist;
- E' die frühe diastolische mitralanuläre Geschwindigkeit ist;
- A die späte diastolische transmitrale Fließgeschwindigkeit ist;
- A' die späte diastolische mitralanuläre Geschwindigkeit ist;
- S die pulmonalvenöse systolische Spitzengeschwindigkeit ist;
- D die pulmonalvenöse frühdiastolische Spitzengeschwindigkeit ist;
- Vp die Geschwindigkeit der Fließprogression ist;
- Gmean und Gmax der mittlere und der maximale transvalvuläre Druckgradient sind;
- VTI das Geschwindigkeits-Zeit-Integral ist;
- S' die systolische annuläre Spitzengeschwindigkeit ist.

6. Verfahren nach einem der vorhergehenden Ansprüche, wobei während des Erfassungsschritts (a) die unfokussierten Ultraschallwellen in mehreren Serien von aufeinanderfolgenden unfokussierten Ultraschallwellen übertragen werden, wobei die aufeinanderfolgenden unfokussierten Ultraschallwellen jeder Serie jeweils unterschiedliche Ausbreitungsrichtungen haben, und der Abbildungsschritt (b) das Synthetisieren eines 3D-Bildes durch synthetische Ultraschallabbildung aus den jeweiligen Rohdaten, die den aufeinanderfolgenden unfokussierten Ultraschallwellen jeder Serie entsprechen, umfasst.

7. Vorrichtung zur 4D-Abbildung eines Herzens eines Lebewesens, wobei die Vorrichtung wenigstens eine 2D-Array-Ultraschallsonde (2) und ein Steuersystem (3, 4) umfasst, das dazu konfiguriert ist:

(a) unfokussierte Ultraschallwellen in dem Herzen durch die 2D-Array-Ultraschallsonde (2) zu übertragen und Rohdaten aus rückgestreuten Ultraschallwellen durch die 2D-Array-Ultraschallsonde (2) zu erfassen,

(b) eine Sequenz von 3D-Bildern aus den Rohdaten zu erzeugen, wobei die Sequenz von 3D-Bildern eine Animation bildet, die Bewegungen eines abgebildeten Volumens zeigt, welches wenigstens einen Teil des Herzens enthält,

(c) eine 3D-Kartographie wenigstens eines Parameters automatisch zu berechnen, der mit der Blutgeschwindigkeit und der Gewebegeschwindigkeit in dem abgebildeten Volumen zusammenhängt, basierend auf der Sequenz von 3D-Bildern

(d) wenigstens einen betrachteten Punkt (13, 14) mit einer vorbestimmten Eigenschaft in der Sequenz von 3D-Bildern automatisch zu lokalisieren, wobei das Steuersystem (3, 4) dazu konfiguriert ist, den wenigstens einen betrachteten Punkt ausschließlich auf der Grundlage der 3D-Kartographie und des zeitlichen Profils automatisch zu lokalisieren;

(e) an dem wenigstens einen betrachteten Punkt (13, 14) wenigstens eine Geschwindigkeit automatisch zu bestimmen, die aus der Blutgeschwindigkeit und der Gewebegeschwindigkeit gewählt wird, und einen vorbestimmten Quantifizierungsparameter zu berechnen, der die wenigstens eine Geschwindigkeit einbezieht, wobei das Steuersystem (3, 4) dazu konfiguriert ist, die wenigstens eine Geschwindigkeit an dem wenigstens einen betrachteten Punkt ausschließlich basierend auf der 3D-Kartographie und ihrem zeitlichen Profil automatisch zu bestimmen, wobei der Quantifizierungsparameter umfasst:

- entweder eine Spitzengeschwindigkeit des Blutes in einem bestimmten anatomischen Bereich und das Steuersystem (3, 4), das dazu konfiguriert ist, automatisch den betrachteten Punkt (13) als einen Punkt maximaler Blutgeschwindigkeit in dem anatomischen Bereich und in wenigstens einem Teil der Sequenz von 3D-Bildern automatisch zu lokalisieren;

- oder eine Spitzengeschwindigkeit des Gewebes in einem bestimmten anatomischen Bereich und das Steuersystem (3, 4), das dazu konfiguriert ist, den anatomischen Bereich in der Folge von 3D-Bildern automatisch zu lokalisieren und den betrachteten Punkt als einen Punkt maximaler Gewebegeschwindigkeit in dem anatomischen Bereich in der Folge von 3D-Bildern automatisch zu lokalisieren.

8. Vorrichtung nach Anspruch 7, wobei der Quantifizierungsparameter gewählt ist aus E, A, E', A', S, D, Vp, S', E/A, E/E', E/E', E'/A', S, S/D, VTI, Gmean und Gmax, wobei:

- E die frühe diastolische transmitrale Fließgeschwindigkeit ist;
- E' die frühe diastolische mitralanuläre Geschwindigkeit ist;
- A die späte diastolische transmitrale Fließgeschwindigkeit ist;
- A' die späte diastolische mitralanuläre Geschwindigkeit ist;
- S die pulmonalvenöse systolische Spitzengeschwindigkeit ist;
- D die pulmonalvenöse frühdiastolische Spitzengeschwindigkeit ist;
- Vp die Geschwindigkeit der Fließprogression ist;
- Gmean und Gmax der mittlere und der maximale transvalvuläre Druckgradient sind;
- VTI das Geschwindigkeit-Zeit-Integral ist;
- S' die systolische ringförmige Spitzengeschwindigkeit ist.

9. Vorrichtung nach einem der Ansprüche 7 bis 8, wobei das Steuersystem (3, 4) dazu konfiguriert ist, die unfokussierten Ultraschallwellen in mehreren Serien von aufeinanderfolgenden unfokussierten Ultraschallwellen zu senden, wobei die aufeinanderfolgenden unfokussierten Ultraschallwellen jeder Serie jeweils unterschiedliche Ausbreitungsrichtungen haben, und das Steuersystem (3, 4) dazu konfiguriert ist, ein 3D-Bild durch synthetische Ultraschallabbildung aus den jeweiligen Rohdaten, die den aufeinanderfolgenden unfokussierten Ultraschallwellen jeder Serie entsprechen, zu synthetisieren.

10. Vorrichtung nach einem der Ansprüche 7 bis 9, wobei das Steuersystem (3, 4) dazu konfiguriert ist, die unfokussierten Ultraschallwellen als divergente Ultraschallwellen zu übertragen.

**11.** Vorrichtung nach einem der Ansprüche 7 bis 10, wobei das Steuersystem (3, 4) dazu konfiguriert ist, die unfokussierten Ultraschallwellen über eine Dauer von wenigstens einem Teil des Herzzyklus und weniger als 10 Herzzyklen zu übertragen.

**12.** Vorrichtung nach einem der Ansprüche 7 bis 11, wobei das Steuersystem (3, 4) dazu konfiguriert ist, die unfokussierten Ultraschallwellen über eine Dauer von 1s bis 10s zu übertragen.

**Revendications**

**1.** Procédé pour l'imagerie en quatre dimensions, 4D, d'un coeur d'un être vivant, ledit procédé comportant au moins les étapes suivantes :

(a) une étape d'acquisition dans laquelle des ondes ultrasonores non focalisées sont émises dans le coeur par une sonde ultrasonore à réseau à deux dimensions, 2D (2) et des données brutes provenant d'ondes ultrasonores rétrodiffusées sont acquises par ladite sonde ultrasonore à réseau 2D (2) ;
(b) une étape d'imagerie dans laquelle une séquence d'images en trois dimensions, 3D, est générée à partir desdites données brutes, ladite séquence d'images en 3D formant une animation montrant des mouvements d'un volume imagé comportant au moins une partie du coeur ;
(c) une étape de calcul de vitesse dans laquelle une cartographie en 3D d'au moins un paramètre se rapportant à la vitesse du sang et à la vitesse des tissus est automatiquement calculée dans ledit volume imagé et au cours du temps, sur la base de ladite séquence d'images en 3D ;
(d) une étape de localisation dans laquelle au moins un point d'intérêt ayant une propriété prédéterminée est automatiquement localisé dans ladite séquence d'images en 3D, dans lequel ledit au moins un point d'intérêt est automatiquement localisé sur la base uniquement de ladite cartographie en 3D et de son profil temporel ;
(e) une étape de quantification dans laquelle au moins une vitesse choisie entre la vitesse du sang et la vitesse des tissus est automatiquement déterminée au niveau dudit au moins un point d'intérêt et un paramètre de quantification prédéterminé est automatiquement calculé, impliquant ladite au moins une vitesse, dans lequel ladite au moins une vitesse est automatiquement déterminée au niveau dudit au moins un point d'intérêt sur la base uniquement de ladite cartographie en 3D et de son profil temporel ;
dans lequel le paramètre de quantification implique :

- soit un pic de vitesse du sang dans une certaine zone anatomique et ladite étape de localisation (d) comporte la localisation, de manière automatique, dudit point d'intérêt (13) comme étant un point de vitesse du sang maximale dans ladite zone anatomique et dans au moins une partie de la séquence d'images en 3D ;
- soit un pic de vitesse des tissus dans une certaine zone anatomique et ladite étape de localisation (d) comporte la localisation, de manière automatique, de ladite zone anatomique dans la séquence d'images en 3D et la localisation, de manière automatique, dudit point d'intérêt comme étant un point de vitesse des tissus maximale dans ladite zone anatomique dans la séquence d'images en 3D.

**2.** Procédé selon la revendication 1, dans lequel le paramètre de quantification implique une variation temporelle du paramètre se rapportant à la vitesse dans une certaine zone anatomique et ladite étape de localisation (d) comporte la localisation, de manière automatique, dudit point d'intérêt (13) comme étant un point de vitesse du sang maximale dans ladite zone anatomique et dans au moins une partie de la séquence d'images en 3D.

**3.** Procédé selon la revendication 1, dans lequel le paramètre de quantification implique une intégrale temporelle du paramètre se rapportant à la vitesse dans une certaine zone anatomique et ladite étape de localisation (d) comporte la localisation, de manière automatique, dudit point d'intérêt (13) comme étant un point de vitesse du sang maximale dans ladite zone anatomique et dans au moins une partie de la séquence d'images en 3D.

**4.** Procédé selon la revendication 1, dans lequel le paramètre de quantification implique une intégrale spatiale du paramètre se rapportant à la vitesse dans une certaine zone anatomique et ladite étape de localisation (d) comporte la localisation, de manière automatique, dudit point d'intérêt (13) comme étant un point de vitesse du sang maximale dans ladite zone anatomique et dans au moins une partie de la séquence d'images en 3D.

**5.** Procédé selon l'une quelconque des revendications précédentes, dans lequel le paramètre de quantification déterminé à ladite étape de quantification est choisi parmi E, A, E', A', S, D, Vp, S', E/A, E/E', E/E', E'/A', S, S/D, VTI, Gmean et Gmax où :

- E est la vitesse d'écoulement transmitral diastolique précoce ;
- E' est la vitesse annulaire mitrale diastolique précoce ;
- A est la vitesse d'écoulement transmitral diastolique tardive ;
- A' est la vitesse annulaire mitrale diastolique tardive ;
- S est le pic de vitesse systolique veineuse pulmonaire ;
- D est le pic de vitesse diastolique précoce veineuse pulmonaire ;
- Vp est la vitesse de progression d'écoulement ;
- Gmean et Gmax sont les gradients moyen et maximal de pression transvalvulaire ;
- VTI est l'intégrale temporelle de vitesse ;
- S' est le pic de vitesse annulaire systolique.

6. Procédé selon l'une quelconque des revendications précédentes, dans lequel au cours de ladite étape d'acquisition (a), lesdites ondes ultrasonores non focalisées sont émises en plusieurs séries d'ondes ultrasonores non focalisées successives, les ondes ultrasonores non focalisées successives de chaque série ayant des directions de propagation respectivement différentes, et ladite étape d'imagerie (b) comporte la synthèse d'une image en 3D par imagerie synthétique par ultrasons à partir des données brutes respectives correspondant auxdites ondes ultrasonores non focalisées successives de chaque série.

7. Appareil pour l'imagerie en 4D d'un coeur d'un être vivant, ledit appareil comportant au moins une sonde ultrasonore à réseau 2D (2) et un système de commande (3, 4) configuré pour :

(a) émettre des ondes ultrasonores non focalisées dans le coeur par le biais de ladite sonde ultrasonore à réseau 2D (2) et acquérir des données brutes provenant d'ondes ultrasonores rétrodiffusées par le biais de ladite sonde ultrasonore à réseau 2D (2),
(b) générer une séquence d'images en 3D à partir desdites données brutes, ladite séquence d'images en 3D formant une animation montrant des mouvements d'un volume imagé comportant au moins une partie du coeur,
(c) calculer automatiquement une cartographie en 3D d'au moins un paramètre se rapportant à la vitesse du sang et à la vitesse des tissus dans ledit volume imagé, sur la base de ladite séquence d'images en 3D,
(d) localiser automatiquement au moins un point d'intérêt (13, 14) ayant une propriété prédéterminée dans ladite séquence d'images en 3D, dans lequel ledit système de commande (3, 4) est configuré pour localiser automatiquement ledit au moins un point d'intérêt sur la base uniquement de ladite cartographie en 3D et de son profil temporel ;
(e) déterminer automatiquement au moins une vitesse choisie entre la vitesse du sang et la vitesse des tissus au niveau dudit au moins un point d'intérêt (13, 14) et calculer automatiquement un paramètre de quantification prédéterminé impliquant ladite au moins une vitesse, dans lequel ledit système de commande (3, 4) est configuré pour déterminer automatiquement ladite au moins une vitesse au niveau dudit au moins un point d'intérêt sur la base uniquement de ladite cartographie en 3D et de son profil temporel,
dans lequel le paramètre de quantification implique :

- soit un pic de vitesse du sang dans une certaine zone anatomique et ledit système de commande (3, 4) configuré pour localiser automatiquement ledit point d'intérêt (13) comme étant un point de vitesse du sang maximale dans ladite zone anatomique et dans au moins une partie de la séquence d'images en 3D ;
- soit un pic de vitesse des tissus dans une certaine zone anatomique et ledit système de commande (3, 4) configuré pour localiser automatiquement ladite zone anatomique dans la séquence d'images en 3D et localiser automatiquement ledit point d'intérêt comme étant un point de vitesse des tissus maximale dans ladite zone anatomique dans la séquence d'images en 3D.

8. Appareil selon la revendication 7, dans lequel le paramètre de quantification est choisi parmi E, A, E', A', S, D, Vp, S', E/A, E/E', E/E', E'/A', S, S/D, VTI, Gmean et Gmax où :

- E est la vitesse d'écoulement transmitral diastolique précoce ;
- E' est la vitesse annulaire mitrale diastolique précoce ;
- A est la vitesse d'écoulement transmitral diastolique tardive ;
- A' est la vitesse annulaire mitrale diastolique tardive ;
- S est le pic de vitesse systolique veineuse pulmonaire ;
- D est le pic de vitesse diastolique précoce veineuse pulmonaire ;
- Vp est la vitesse de progression d'écoulement ;
- Gmean et Gmax sont les gradients moyen et maximal de pression transvalvulaire ;

- VTI est l'intégrale temporelle de vitesse ;
- S' est le pic de vitesse annulaire systolique.

9. Appareil selon l'une quelconque des revendications 7 à 8, dans lequel ledit système de commande (3, 4) est configuré pour émettre lesdites ondes ultrasonores non focalisées en plusieurs séries d'ondes ultrasonores non focalisées successives, les ondes ultrasonores non focalisées successives de chaque série ayant des directions de propagation respectivement différentes, et ledit système de commande (3, 4) est configuré pour synthétiser une image en 3D par imagerie synthétique par ultrasons à partir des données brutes respectives correspondant auxdites ondes ultrasonores non focalisées successives de chaque série.

10. Appareil selon l'une quelconque des revendications 7 à 9, dans lequel ledit système de commande (3, 4) est configuré pour émettre lesdites ondes ultrasonores non focalisées sous la forme d'ondes ultrasonores divergentes.

11. Appareil selon l'une quelconque des revendications 7 à 10, dans lequel ledit système de commande (3, 4) est configuré pour émettre lesdites ondes ultrasonores non focalisées pendant une durée d'au moins une partie d'un cycle cardiaque et de moins de 10 cycles cardiaques.

12. Appareil selon l'une quelconque des revendications 7 à 11, dans lequel ledit système de commande (3, 4) est configuré pour émettre lesdites ondes ultrasonores non focalisées pendant une durée comprise entre 1 s et 10 s.

## FIG. 1

## FIG. 2

**FIG. 3**

**FIG. 4**

**FIG. 5**

FIG. 6

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2015366532 A1
- US 2016140730 A1
- US 2013144161 A1
- US 2011208056 A1
- WO 2017069699 A1

### Non-patent literature cited in the description

- **M. TANTER ; M. FINK.** Ultrafast imaging in biomedical ultrasound. *IEEE Trans. Ultrason. Ferroelectr. Freq. Control,* January 2014
- **J. PROVOST et al.** 3D ultrafast ultrasound imaging in vivo. *Phys. Med. Biol.,* 01 October 2014, vol. 59 (19
- **MONTALDO, G. ; TANTER, M. ; BERCOFF, J. ; BENECH, N. ; FINK, M.** Coherent plane-wave compounding for very high frame rate ultrasonography and transient elastography. *IEEE Trans. Ultrason. Ferroelectr. Freq. Control,* 2009, vol. 56, 489-506
- Synthetic aperture tissue and flow ultrasound imaging. **NIKOLOV, S.I.** Orsted-DTU. Technical University of Denmark, 2001
- **NIKOLOV, S.I. ; KORTBEK, J. ; JENSEN, J.A.** Practical applications of synthetic aperture imaging. *2010 IEEE Ultrasonics Symposium (IUS). Presented at the 2010 IEEE Ultrasonics Symposium (IUS),* 2010, 350-358
- **LOCKWOOD, G.R. ; TALMAN, J.R. ; BRUNKE, S.S.** Real-time 3-D ultrasound imaging using sparse synthetic aperture beamforming. *IEEE Trans. Ultrason. Ferroelectr. Freq. Control,* 1998, vol. 45, 980-988
- **PAPADACCI, C. ; PERNOT, M. ; COUADE, M. ; FINK, M. ; TANTER, M.** High-contrast ultrafast imaging of the heart. *IEEE transactions on ultrasonics, ferroelectrics, and frequency control,* 2014, vol. 61, 288-301
- **KASAI, C. ; NAMEKAWA, K. ; KOYANO, A. ; OMOTO, R.** Real-Time Two-Dimensional Blood Flow Imaging Using an Autocorrelation Technique. *IEEE Trans. Sonics Ultrason,* 1985, vol. 32, 458-464
- **DEMENÉ, C. et al.** Spatiotemporal Clutter Filtering of Ultrafast Ultrasound Data Highly Increases Doppler and Ultrasound Sensitivity. *IEEE transactions on medical imaging,* 2015, vol. 34, 2271-2285